# EUROPÄISCHE PATENTSCHRIFT

(11) **EP 0 681 678 B1**
(45) Veröffentlichungstag und Bekanntmachung des Hinweises auf die Patenterteilung: **03.01.2001**
(21) Anmeldenummer: 94929517.4
(22) Anmeldetag: 06.10.1994
(51) Int. Cl.: F41A 3/54, F41A 3/26, F41A 9/32, F41A 9/33

(54) **SELBSTLADE-GRANATWERFER**
SELF-LOADING GRENADE LAUNCHER
LANCE-GRENADES A CHARGEMENT AUTOMATIQUE

(30) Priorität: 08.10.1993 DE 4334412
(43) Veröffentlichungstag der Anmeldung: 15.11.1995
(60) Teilanmeldung: 98113916.5
(73) Patentinhaber: HECKLER & KOCH GMBH, D-78727 Oberndorf (DE)
(72) Erfinder: WEICHERT, Berthold, D-78658 Zimmern (DE); WÖSSNER, Ernst, D-72172 Sulz (DE); GIELKE, Gerhard, D-78727 Oberndorf (DE); GABLOWSKI, Jürgen, D-78727 Oberndorf (DE)
(74) Vertreter: von Samson-Himmelstjerna, Friedrich R., Dipl.-Phys.
(86) Internationale Anmeldenummer: EP9403307
(87) Internationale Veröffentlichungsnummer: WO9510747

(56) Entgegenhaltungen:
- EP-A- 0 378 976
- DE-A- 2 016 281
- DE-C- 700 629
- GB-A- 629 224
- US-A- 2 960 917
- US-A- 4 942 802

## Beschreibung

Die Erfindung betrifft einen Selbstlade-Granatwerfer.

Ein solcher Granatwerfer ist in der Zeitschrift "International Defense Review", Band 22, Nr, 12/1989 beschrieben. Er weist eine Patronengurt-Zuführeinrichtung auf, die die jeweils vorderste Patrone unmittelbar hinter das Patronenlager fördert. Ein Masseverschluß, der in der Wirkungsweise dem einer Maschinenpistole ähnelt, wird durch eine Schließfederanordnung gegen diese vorderste Patrone gefördert, schiebt sie in das Patronenlager und zündet sie.

Im folgenden werden Begriffe wie "vorne", "hinten", "seitlich" usw. ohne nähere Definition verwendet. Sie beziehen sich stets auf die in horizontaler Feuerstellung befindliche Waffe, wobei "vorne" die Mündung, also das in Schußrichtung vorderste Ende der Waffe, bedeutet.

Um den Granatwerfer feuerbereit zu machen, genügt es, den Masseverschluß gegen die Wirkung der Schließfederanordnung in seine hinterste Lage zu bewegen, in welcher er von einer Abzugseinrichtung festgehalten wird, und den Patronengurt in die Zuführeinrichtung einzulegen.

In diesem einfachen Bewegungsablauf liegt der besondere Vorteil des gattungsbildenden Granatwerfers gegenüber dem bisher am weitesten verbreiteten Selbstlade-Granatwerfer, dem US Mark 19: bei diesem muß nämlich nach dem Einlegen des Gurtes der Verschluß zunächst einmal leer abgeschlagen und dann wieder gespannt werden, da die vorderste Patrone des Gurtes beim ersten Abschlagen des Verschlusses nicht unmittelbar in das Patronenlager, sondern zunächst in eine Übergabeposition gefördert wird, aus welcher sie durch das zweite Abschlagen des Verschlusses in das Patronenlager gefördert und dort gezündet wird.

Dieser komplizierte Ladevorgang führt zu Bedienungsfehlern, aufgrund deren die Waffe nach dem Laden entweder nicht feuerbereit ist oder unzeitig abgefeuert wird.

Der gattungsbildende Granatwerfer, der diesen Nachteil nicht aufweist und baulich recht einfach ist, hat allerdings eine unbefriedigende Betriebs- und Funktionssicherheit.

Ausgehend von diesem Stand der Technik liegt der Erfindung die Aufgabe zugrunde, einen Selbstlade-Granatwerfer zu schaffen, der unter Nutzung der oben beschriebenen Vorzüge des bekannten Granatwerfers eine bessere Betriebssicherheit und eine bessere Funktionssicherheit aufweist.

Diese Aufgabe wird durch die Merkmale des Anspruchs 1 gelöst.

Es ist zwar bereits aus der EP-A-0 378 976 eine Maschinenpistole bekannt, bei der der Spanngriff, der am Verschluß befestigt ist, mit dem Gehäuse über ein Ratschengesperre so verbunden ist, daß der Spanngriff beim Zurückziehen des Verschlusses erst dann wieder nach vorne gleiten kann, wenn der Verschluß eine Mindest-Spannlage erreicht hat; so wird verhindert, daß sich ein Schuß lösen kann, wenn der Schütze versehentlich den Verschluß mittels des Spanngriffes nicht weit genug zurückgezogen hat und dann den Spanngriff losläßt. Bei Erreichen der Mindest-Spannlage wird aber das Ratschengesperre in wirkungsloser Lage blockiert, so daß etwa nach Abfeuern einer zu schwachen Patrone der Verschluß wieder nach vorne schnellen kann, obwohl er seine Mindest-Spannlage nicht erreicht hat. Erst, wenn der Schütze am Spanngriff angreift und mit dessen Zurückziehen beginnt, wird das Ratschengesperre wieder aktiviert, so daß es nur bei der Betätigung des Spanngriffes wirksam ist.

Dagegen ist bei der Erfindung die Sperre zwischen dem Masseverschluß und dem Gehäuse angeordnet und ist ständig wirksam, unabhängig davon, ob dabei der Spanngriff betätigt wird oder nicht.

Der Masseverschluß zündet in bekannter Weise, wie es auch beim gattungsbildenden Granatwerfer der Fall ist, die Patrone, die gerade in das Patronenlager eingeführt wird, während der letzten Phase seiner Vorwärtsbewegung; hierbei wird die kinetische Energie des sich nach vorne bewegenden Masseverschlusses ausgenutzt, um dem einsetzenden Rückstoß der abgefeuerten Patrone entgegenzuwirken und zu verhindern, daß die Patronenhülse vorzeitig aus dem Patronenlager gelangt.

Der Zündzeitpunkt hängt ab von der Geschwindigkeit, mit welcher sich der Masseverschluß nach vorne bewegt. Die erforderliche Geschwindigkeit wird aber nur erreicht, wenn der Masseverschluß bei seinem Rücklauf eine Mindest-Spannlage erreicht und von dieser aus seine Schließbewegung beginnt.

Wenn beim gattungsbildenden Granatwerfer infolge irgendeiner Störung (klemmende Patronenhülse, unvollkommen oder verzögert abbrennende Treibladung, unvollständiges Zurückziehen des Spannhebels etwa nach einem Patronenversager) der Masseverschluß diese Mindest-Spannlage nicht erreicht, dann erfolgt die Zündung der nachfolgenden Patrone zu frühzeitig, bevor sie ausreichend in das Patronenlager eingeführt wurde. Der vorzeitig einsetzende Gasdruck kann die Patronenhülse sprengen und mindestens zu einer Ladehemmung, wahrscheinlich aber auch zur Beschädigung der Waffe führen.

Erfindungsgemäß sieht der Anspruch 1 eine Sperre vor, die in einem solchen Störfall, wenn der Masseverschluß beim Rücklauf nicht die Mindest-Spannlage erreicht, den Vorlauf des Masseverschlusses verhindert. Bei der Wirkung der Sperre wird der Schütze wie bei einer Ladehemmung ohne nähere Prüfung der Ursache der Störung den Spannhebel in gewohnter Weise zurückziehen und dabei'den Verschluß bis hinter die Mindest-Spannlage bewegen, so daß der weitere störungsfreie Betrieb ermöglicht ist. Es wird nicht einmal eine volle Patrone ausgeworfen, sondern die Verschlußbewegung fährt dort fort, wo sie die Sperre angehalten hat.

Somit wird durch die Erfindung das Auftreten einer ernsthaften Ladehemmung oder sogar einer Beschädigung der Waffe vermieden.

Diese Mindest-Spannlage definiert die Position, die der Masseverschluß einnehmen muß, um nicht vorzeitig die jeweils nachgeführte Patrone zu zünden.

Die Auslöselage entspricht der Position, die der Masseverschluß einnimmt, wenn er mittels der Abzugseinrichtung ausgelöst wird. Diese Auslöselage ist so gewählt, daß ein optimaler Zündzeitpunkt sichergestellt ist.

Gemäß der Ausgestaltung des Anspruchs 2 wird nun die Mindest-Spannlage möglichst nahe an diese optimale Auslöselage herangeführt.

Gemäß Anspruch 3 ist die Sperre, die das Abschlagen des Verschlusses verhindert, wenn er die Mindest-Spannlage nicht erreicht hat, als Ratschengesperre ausgebildet, mit einer Reihe von Ratschenzähnen und einer relativ zu diesen bewegten Ratschenklinke. Ratschenzähne sind Zähne, deren eine Flanke sanft und deren andere Flanke steil ansteigt. Vor und hinter der Reihe von Ratschenzähnen ist für die Ratschenklinke ein Raum vorgesehen, in welchem sie ihre Orientierung ändert.

Hierbei sind die Ratschenzähne so angeordnet, daß sie beim Rücklauf des Masseverschlusses mit ihren sanft ansteigenden Flanken gegen die Ratschenklinke laufen.

Beim Rücklauf des Masseverschlusses ist die Ratschenklinke so orientiert, daß sie mit ihrem freien Ende nach hinten weist. Dabei wird sie federnd gegen die Ratschenzähne gedrückt und von deren sanft ansteigenden Flanken weggedrückt.

Kehrt in dieser Phase der Masseverschluß seine Bewegungsrichtung um, dann hintergreift die Ratschenklinke eine steile Flanke eines Ratschenzahnes und sperrt die Bewegung des Masseverschlusses.

Am Ende der Reihe von Ratschenzähnen an einer Stelle des Rücklaufes des Masseverschlusses, der der Mindest-Spannlage entspricht, kommt die Ratschenklinke frei und ändert ihre Orientierung so, daß sie von den steil ansteigenden Flanken der Ratschenzähne wegweist, deshalb in diese nicht eingreifen kann und mithin die Bewegung des Masseverschlusses nicht behindert.

Das Umschalten der Orientierung der Ratschenklinke erfolgt durch eine Nase, die an einer Stelle angebracht ist, die der Mindest-Spannlage entspricht.

Gemäß Anspruch 4 sind die Ratschenzähne zu einer zahnstangenartigen Anordnung zusammengefaßt, die entweder am Masseverschluß oder am Waffengehäuse angebracht ist, während die Ratschenklinke, die mittels einer Federanordnung quer zur Zahnstange ausgerichtet ist, am anderen dieser Elemente sitzt.

Bevorzugt sitzt die Ratschenklinke am Gehäuse, da bei einer Anbringung am Masseverschluß auf sie einwirkende Massenkräfte ihre Funktion beeinträchtigen könnten.

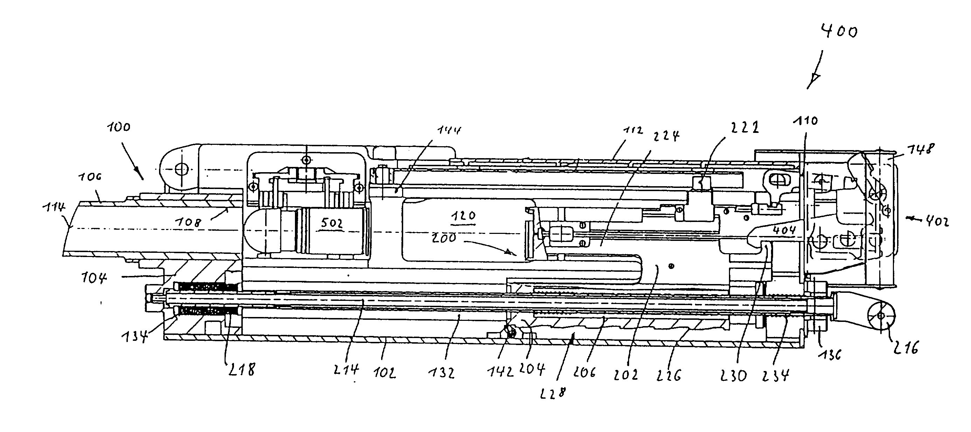

Der Gegenstand der Erfindung ist in der beigefügten Zeichnung noch näher erläutert.

Diese an sich sehr einfache Steuerung hat sich allerdings als unbefriedigend erwiesen. Der Grund hierfür dürfte in der Tatsache liegen, daß sich die Schieber während sehr kurzer Bewegungsstrecken des Masseverschlusses über verhältnismäßig lange Strecken bewegen müssen, so daß ihre unmittelbare Führung über stark abgewinkelte Führungsnuten erfolgen muß, damit zu hohen Kräften führt und etwa bei Verschmutzung nicht mehr zuverlässig erfolgen kann. Außerdem ist der Verschleiß hoch.

Erfindungsgemäß wird dieses Problem mangelnder Funktionssicherheit und Verschleißbeständigkeit durch die Merkmale des Anspruchs 5 gelöst.

Hierbei ist ein gesonderter Kurvenhebel vorgesehen, der sich längs der Bewegungsbahn des Masseverschlusses erstreckt und mit diesem über eine aus Führungskurve und Mitnehmer gebildete Steuerverbindung gesteuert wird. Der Kurvenhebel ist mit den Schiebern über ein Gestänge verbunden.

Somit ist es möglich, günstige Kräfteverhältnisse im Eingriff zwischen Führungskurve und Mitnehmer herzustellen. Außerdem ist es möglich, das Material des Kurvenhebels, der bevorzugt eine Führungsnut aufweist, hinsichtlich besonders geringen Verschleißes zu optimieren. Auch der Mitnehmer, der vorzugsweise aus einer am Masseverschluß angebrachten Rolle besteht, kann optimiert werden, wobei sein Gewicht zu dem ohnehin erforderlichen Gewicht des Masseverschlusses beiträgt und somit unproblematisch ist.

Gemäß Anspruch 6 ist der Kurvenhebel an seinem vorderen Ende schwenkbar gelagert; seine Schwenkbewegung wird hinter seiner Mitte von einem zweiarmigen Umlenkhebel abgegriffen und nach vorne übertragen. Diese auf den ersten Blick umständlich erscheinende Anordnung ermöglicht eine Kraftübertragung vom Kurvenhebel auf die Schieber ohne Übersetzung. Die notwendigen Toleranzen zwischen Mitnehmer und Steuerkurve, die die zuverlässige Funktion der Waffe auch bei Verschmutzung sicherstellen, werden somit ebenfalls nicht übersetzt.

Gemäß Anspruch 7 ist die Hebelsteuerung, die das Gestänge mit den Schiebern verbindet, mittig angeordnet und mit zwei Steuerhebeln symmetrisch ausgebildet. Diese Anordnung ist eine Vorbedingung für die Austauschbarkeit der Schieber und damit für die freie Wahl der Richtung der Zuführung des Patonengurtes.

Der Anspruch 8 befaßt sich mit der näheren Ausbildung der Steuerhebel, die in einem aufklappbaren Deckel gelagert sind und erfindungsgemäß mit einer lösbaren Kupplung versehen sind, mit welcher sie problemlos in und außer Eingriff mit dem Gestänge gelangen, das im Gehäuse der Waffe oberhalb des Masseverschlusses sitzt. Hierbei erfolgt die Steuerung einfacherweise nur über einen der beiden Steuerhebel, der sie seinerseits auf den anderen Steuerhebel überträgt.

Diese Übertragung erfolgt gemäß Anspruch 9 durch eine gelenkige Verbindung zwischen Schenkeln der Steuerhebel. Diese gelenkige Verbindung ist gemäß Anspruch 10 ohne Verwendung irgendeines Zwischenelementes durchgeführt.

Der Eingriff eines jeden Steuerhebels in den ihm zugeordneten Schieber erfolgt gemäß Anspruch 11 in jeweils gleichartiger Weise, was nicht nur eine Herstellungsvereinfachung ermöglicht, sondern eine weitere Vorbedingung für die oben bereits angesprochene Austauschbarkeit der Schieber ist.

Gemäß Anspruch 12 ist jeder Schieber in einer Querführung geführt; diese beide Querführungen sind gleichartig so ausgebildet, daß die beiden Schieber austauschbar sind.

Bei diesem Austausch werden die Schieber nicht nur ausgetauscht, sondern in ihrer Richtung umgekehrt, also umgedreht, so daß die Querführungen zu ihrer Längsachse symmetrisch ausgebildet oder wirksam sein müssen.

Dieser Austausch der Schieber ermöglicht es, die Waffe je nach Bedarf mit der Zuführung von rechts oder links auszubilden, so daß beim Einbau der Waffe etwa im Einstieg eines Hubschraubers oder an der Luke eines Panzerfahrzeuges die Zuführung des Patronengurtes von der optimal geeigneten Seite her erfolgen kann.

Es ist eine weitere Aufgabe der Erfindung, diese Zuführung so auszubilden, daß sie zu diesem Zweck geeignet ist und außerdem mit größter Funktionssicherheit den Patronengurt nachführt und entgurtet.

Diese Aufgabe wird durch die Merkmale des Anspruchs 13 gelöst.

Die einzelnen Patronen sind etwa in der Längsmitte der Patronenhülse von einem wie eine Rohrschelle ausgebildeten Gurtglied reibschlüssig umgriffen, das auf der einen Seite einen gelenkig am Gurtglied befestigten, mit einem Kopf versehenen Zapfen und auf der gegenüberliegenden Seite einen abstehenden, bügelförmigen Abschnitt mit einem Langloch aufweist. Das Langloch ist am vorderen Ende erweitert und so bemessen, daß der Kopf des Zapfens der benachbarten Patrone die Erweiterung des Langloches, aber nicht dessen übrigen Abschnitt durchsetzen kann, in dem der Hals des Zapfens geführt ist. Mittels der Zapfen-Langloch-Verbindung sind die einzelnen Gurtglieder gelenkig aneinandergehängt.

Bei der Schieberanordnung, die in Anspruch 13 beschrieben ist, bewegen sich beim Vorlauf des Masseverschlusses beide Schieber gegenläufig jeweils von außen aus einer Ausgangslage nach innen, wobei die Innenklinke des ersten Schiebers die erste Patrone hintergreift und bis vor das Patronenlager fördert. Gleichzeitig bewegt sich der zweite Schieber aus seiner Ausgangslage gegenläufig und gelangt mit der an ihm angebrachten festen Stütze bis neben die erste Patrone, wo diese Stütze verhindert, daß die Patrone über ihre Lage hinter dem Patronenlager hinausrutscht. Nun ist der Masseverschluß hinter dieser Patrone angelangt und schiebt sie in das Patronenlager ein, wobei das Gurtglied auf dem Rand des Patronenlagers aufsitzt und auf der Patrone nach hinten rutscht. Bei der Zündung der Patrone befinden sich das Gurtglied und der hintere, von ihm umschlossene Teil der Patronenhülse außerhalb des Patronenlagers.

Beim Rücklauf schiebt die abgefeuerte Patronenhülse den Masseverschluß nach hinten bis zu einer Position hinter der Patronengurt-Zuführeinrichtung, wo die Patronenhülse auf einen üblichen Ausstoßer auftrifft und durch ein einziges, seitliches Fenster ausgeworfen wird, gleichgültig, ob die Zuführung des Patronengurtes von rechts oder links erfolgt.

Gleichzeitig bewegen sich die beiden Schieber in ihre jeweilige Ausgangslage zurück, wobei die Innenklinke des ersten Schiebers über die nachfolgende Patrone hinwegschwenkt und die Stütze des zweiten Schiebers sich nach außen bewegt, um das Ausziehen der Patronenhülse nicht zu stören. Gleichzeitig schiebt die an diesem zweiten Schieber angeordnete Schwenkklinke die nachfolgende Patrone weiter in eine Position, in welcher sie von der genannten Innenklinke beim nächsten Vorlauf des Masseverschlusses weitergeführt wird.

Wie ersichtlich, erfolgt die Nachführung einer jeden Patrone in zwei aufeinanderfolgenden Schritten jeweils beim Vorlauf und Rücklauf des Masseverschlusses, so daß zu hohe Beschleunigungen und somit Massekräfte vermieden sind.

Es wird ausdrücklich darauf hingewiesen, daß hier und in den Ansprüchen jeweils nur von einer Klinke, Stütze usw. die Rede ist, aber bevorzugt mehrere, am besten zwei, derartige Elemente in Längsrichtung der Waffe (quer zum Patronengurt) nebeneinanderliegend angeordnet sind, um die Patrone bei allen ihren Bewegungen stets zur Längsrichtung der Waffe ausgerichtet zu halten.

Somit ist die Waffe geeignet, auch einen Patronengurt der genannten Art störungsfrei aufzunehmen, bei dem infolge der Befestigung der Gurtglieder untereinander diese gegeneinander verschwenkbar sind.

Gemäß Anspruch 14 sitzt am innenliegenden Ende des ersten Schiebers ein fester Anschlag ähnlich der Stütze am Außenende des zweiten Schiebers; diese Stütze hat die Aufgabe, zu verhindern, daß der bei offenem Verschluß nachgeführte Patronengurt mit seiner dann vordersten Patrone über die Position hinausrutscht, die diese dann einnehmen soll, wenn sie von der Innenklinke erfaßt und nachgeführt werden soll.

Es hat sich bei Versuchen herausgestellt, daß der Patronengurt beim Feuern sehr heftige, peitschenartige Bewegungen ausführt und Erschütterungen erfährt, die das Einführen der hinter das Patronenlager nachgeführten, auf dieses ausgerichteten, vordersten Patrone beeinträchtigen kann.

Um diesem Nachteil entgegenzuwirken, wird erfindungsgemäß vorgeschlagen, unmittelbar vor dem bzw. während des Einführens der Patrone in das Patronenlager den Patronengurt, von dem diese Patrone bereits abgetrennt bzw. ausgegurtet ist, von dieser Patrone ein wenig zurückzuziehen; der Patronengurt kann nun nicht mehr durch seine unvermeidlichen Bewegungen gegen die Patrone anschlagen, die gerade in das Patronenlager eingeführt wird.

Es ist gleichzeitig auch von Vorteil, die am zweiten Schieber angebrachte Stütze von der Patrone weg zurückzufahren, damit sie nicht vom Kopf des Masseverschlusses gewaltsam zur Seite gedrückt wird und dabei unnötig hohem Verschleiß ausgesetzt ist.

Während man den genannten Störungen bisher dadurch begegnete, daß man die Patrone mit großem seitlichem Spiel vor dem Patronenlager anordnete, wird bei der Erfindung die Patrone exakt vor dem Patronenlager gehalten, bis der Einführvorgang einsetzt, und erst dann von ihren seitlichen Führungen freigegeben. Im Ergebnis erzielt die Erfindung somit eine sehr hohe Funktionssicherheit, ungeachtet der Lage und Ausrichtung der Waffe oder der auf diese einwirkenden Beschleunigungen. Die erfindungsgemäße Waffe kann somit auch auf einem durch das Gelände fahrenden Fahrzeug während der Fahrt geschossen werden, ohne daß Fahrbahnstöße die Nachladefunktion beeinträchtigen.

Gemäß der bevorzugten Ausgestaltung nach Anspruch 15 wird diese Freigabe der Patrone dadurch bewirkt, daß die Bewegungsumkehr der Schieber nicht erst beim Abschuß, also in vorderster Stellung des Masseverschlusses, sondern schon ein wenig früher einsetzt, so daß eine am ersten Schieber angebrachte weitere Außenklinke, die zu diesem Zeitpunkt gegen Abkippen gesperrt ist, den Patronengurt zurückzieht und sich die Stütze von der gerade in das Patronenlager eingeführten Patrone wegbewegt.

Um diese Außenklinke zu sperren, also um ihr Abkippen zu verhindern, ist gemäß Anspruch 16 der zweite Schieber so ausgebildet, daß er die Außenklinke übergreift und somit sperrt. Somit gelingt es, diese Sperrfunktion ohne zusätzliches Bauteil in genauer Zuordnung zum Bewegungsablauf der beiden Schieber zu realisieren.

Wie bereits oben erläutert, befindet sich die vorderste Patrone des Patronengurtes in einer Zwischenposition, wenn sich der Masseverschluß in seiner hintersten Lage (Auslöselage) befindet. In dieser Zwischenposition wird die vorderste Patrone von der Schwenkklinke des zweiten Schiebers hintergriffen und abgestützt.

Beim Einlegen des Patronengurtes ist aber der diese Schwenkklinke tragende Deckel hochgeklappt.

Außerdem liegt in der Feuerbereitschaft die vorderste Patrone ständig gegen diese Schwenkklinke an, so daß sie allen vom Patronengurt ausgeübten Massenkräften standhalten muß.

Um die genaue Lage des Patronengurtes bei dessen Einlegen und bei offenem Deckel zu fixieren, und um die Massekräfte des Patronengurtes aufzunehmen, ist gemäß Anspruch 18 unterhalb des zugeführten Patronengurtes ein wie eine Klinke wirksamer, nach oben abgefederter Sperrhebel angeordnet, gegen dessen zur Längsmitte der Waffe hin weisendes, abstehendes Ende die vorderste Patrone mit ihrer Außenseite in der genannten Zwischenposition anliegt.

Wird der Gurt weitergefördert, dann weicht der Sperrhebel der nachfolgenden Patrone nach unten aus, ohne sie zu behindern, und taucht dann, wenn sie sich in der Zwischenlage - befindet, wieder nach oben, um wieder als Abstützung wirksam zu sein. Somit wird nicht nur ein Anschlag zum präzisen Einlegen des Patronengurtes geschaffen, sondern auch das Ausleiern oder gar Abbrechen der Schwenkklinke oder mit ihr verbundener Bauteile verhindert.

Die für den erfindungsgemäßen Granatwerfer verwendete Patrone ist eine Randpatrone, also eine Patrone mit einem radial überstehenden Rand. Außerdem trägt diese Patrone ein Gurtglied. Liegt eine solche Patrone auf einer ebenen Fläche, dann ist die Längsachse der Patrone zu dieser Fläche geneigt. Das Bewegen einer solchen Patrone exakt parallel zu ihrer Längsachse ist somit problematisch.

Angesichts dieser Problemlage zielt die Erfindung darauf ab, das exakte und damit besonders störungsfreie Einführen der Patrone in das Patronenlager zu ermöglichen.

Diese Aufgabe wird durch die Merkmale des Anspruchs 19 gelöst.

Hierbei ist unterhalb des Patronenlagers ein Führungstisch ausgebildet, auf dem der herangeförderte Patronengurt aufliegt und glatt und störungsfrei bis auf eine Patronenauflage nachgefördert werden kann.

Wenn der Ausgurtvorgang erfolgt und die vorderste Patrone in das Patronenlager eingeführt wird, dann weicht die Patronenauflage so nach unten aus, daß sich die an ihrem Boden vom Kopf des Masseverschlusses erfaßte Patrone auf diesen ausrichten kann und somit einwandfrei in das Patronenlager eingeführt wird.

Die Patronenspitze verbleibt dabei stets auf der Höhe der Mitte des Patronenlagers; lediglich der hintere Teil taucht beim Ausweichen der Patronenauflage soweit ab, daß die Achse der Patrone voll auf die Seelenachse ausgerichtet ist.

Um sicherzustellen, daß die Patronenauflage nicht zur Unzeit nachgibt, ist sie gemäß Anspruch 20 durch die Bewegung des Masseverschlusses gesteuert und wird von diesem erst dann ausgelöst, kurz bevor oder wenn er die vorderste Patrone ausgurtet und dabei in das Patronenlager einführt.

Die Patronenauflage trägt die verhältnismäßig schwere Patrone mindestens für eine kurze Zeit, wobei auf die Waffe einwirkende Stöße die Belastung der Patronenauflage vervielfachen können. Eine Federbelastung allein ist daher wohl nicht ausreichend, um sicherzustellen, daß die Patronenauflage erst dann nachgibt, wenn sie vom Masseverschluß angesteuert wird.

Um diesem Problem abzuhelfen, ist gemäß Anspruch 21 eine Arretierung vorgesehen, die die Patronenauflage in ihrer normalen Lage festhält. Die Arretierung wird vom Masseverschluß gelöst, so daß das Lösen der Arretierung stets zuverlässig auf das Absenken der Patronenauflage, das ebenfalls vom Masseverschluß gesteuert ist, abgestimmt ist.

Die Patronenauflage kann als Platte ausgebildet werden, ist aber gemäß Anspruch 22 oder 23 als Anordnung aus mindestens einem Querfinger ausgebildet, dessen Masse verhältnismäßig gering ist, so daß sein rasches Ausweichen und Zurückschwenken keine Störungen in der Verschlußbewegung und insbesondere keinen hohen Verschleiß erbringt.

Es ist eine allgemeine Regel, daß man Schußwaffen aller Art nicht leer, also ohne Patrone oder Pufferpatrone im Patronenlager, abschlagen lassen soll.

Andererseits ist es bei der Ausbildung an den Waffen erforderlich, das Schießen, Ladegriffe usw. häufig zu üben.

Um die Beschädigung des erfindungsgemäßen Granatwerfers zu vermeiden, die er durch das leere Abschlagen des Masseverschlusses erleiden könnte, ist gemäß Anspruch 24 eine Pufferfedereinrichtung vorgesehen, die den Masseverschluß in der letzten Phase des Vorlaufes abfängt und abbremst.

Diese Pufferfedereinrichtung ist so angeordnet, daß sie beim normalen Schießvorgang nicht in Wirkung tritt, da dann der Masseverschluß allenfalls bis zu dem ein Stück weit aus dem Patronenlager herausstehenden Boden der Patrone vorlaufen kann, nicht aber bis zur Pufferfedereinrichtung.

Bevorzugt (Anspruch 25) sind zwei sich parallel zur Waffenlängsachse erstreckende Federführungsstäbe für die Schließfedern vorgesehen, die den Masseverschluß jeweils in einer Längsbohrung durchsetzen. Am vorderen Ende dieser Federführungsstäbe ist jeweils eine Pufferfeder angeordnet, die gegen das vordere Ende des Gehäuses abgestützt ist und bevorzugt teilweise in einer Bohrung sitzt, damit ein hinlänglicher Raum für deren Aufnahme geschaffen ist.

Die Anordnung zweier Pufferfedern liefert wie auch die Anordnung zweier Schließfedern eine Redundanz von Teilen, die im Falle des Brechens einer Feder einen weiteren Betrieb der Waffe, wenn auch vielleicht unter Störungen, ermöglicht.

Die Pufferfedern können gegebenenfalls nur für Ausbildungszwecke eingebaut sein, vor dem Einsatz der Waffe aber ausgebaut werden.

Bei einer herkömmlichen Selbstladewaffe ist in der Regel der Verschluß in Nuten und Stegen geführt, die im Inneren des Gehäuses ausgebildet sind. Diese Gleitflächen münden in die Auswurföffnung und die Öffnung zum Zuführen des Patronengurtes ein und können von dort her leicht verschmutzen, was zu Funktionsstörungen führen kann.

Außerdem müssen die Außenwände des Gehäuses, die diese Gleitflächen bilden, hinlänglich steif und somit entsprechend schwer ausgebildet sein.

Außerdem ist es aufwendig, die mit großem gegenseitigem Abstand angeordneten, einander gegenüberliegenden Gleitflächen mit der ausreichenden Genauigkeit herzustellen.

Um die Funktionssicherheit des erfindungsgemäßen Granatwerfers bei gleichzeitig verringertem Bauaufwand zu verbessern, ist gemäß Anspruch 26 der Masseverschluß nicht an seiner Außenfläche geführt, sondern von einem Längskanal durchsetzt, mit dem er auf einer im Gehäuse ortsfest angebrachten Längsführung verschieblich sitzt.

Die verhältnismäßig geringen Abmessungen der Längsführung und des Längskanales ermöglichen eine einfache Fertigung bei hinlänglicher Maßhaltigkeit. Die Gehäuseseitenwände müssen, wenn sie nicht sonstige Funktionen erfüllen sollen, lediglich als Abdeckung ausgebildet sein und können entsprechend leicht oder aus entsprechend weniger stabilem Material, etwa Kunststoff, gebildet sein.

Am wichtigsten ist aber der Umstand, daß diese Führungsanordnung im Inneren des Gehäuses weit von Öffungen in diesem entfernt ist, durch die Schmutz ins Innere des Gehäuses gelangen kann.

Bei geeigneter Anordnung der Längsführung können deren Enden in Bereichen des Gehäuses angeordnet sein, in denen keine funktionswesentlichen Teile sitzen; Schmutz, der vom bewegten Masseverschluß zu den Enden der Längsführung geschoben wird, kann sich dort ansammeln, ohne die Funktionssicherheit der Waffe zu beeinträchtigen. Somit ist der Betrieb der Waffe auch in Staub und Schlamm über lange Zeit hinweg möglich, ohne daß eine Reinigung der Waffe unbedingt erforderlich wäre.

Eine besonders kostengünstige, zugleich verschmutzungssichere und somit funktionssichere Ausgestaltung liegt gemäß Anspruch 27 in der Verwendung eines Rundstabes als Längsführung, der in einer Spiel-Paßbohrung im Masseverschluß geführt ist.

Der Rundstab kann Ringnuten zur Aufnahme von Schmierstoff, Gleitringen oder Schmutz aufweisen.

Eine einfache Abstützung des Verschlusses, wie sie etwa durch den Eingriff des Mitnehmers in die Führungsnut des Kurvenhebels gegeben ist, reicht völlig aus, um das Verdrehen des Masseverschlusses um den Rundstab zu verhindern.

Das Gehäuse der gattungsbildenden Selbstladewaffe bietet große Probleme, auch wenn es nicht als Längsführung für den Masseverschluß dienen soll: die Ausführung als Stahl- oder Leichtmetall-Schmiedestück, das spanend nachbearbeitet ist, ist schwer und außerdem sehr kostenspielig. Eine Schweißkonstruktion aus Zuschnitten oder Stanz- und Biegeteilen ist zwar leichter und billiger, aber noch immer schwierig verzugsfrei unf maßhaltig herzustellen. Außerdem weist ein geschweißtes Gehäuse feine, unzugängliche Nuten auf, die Ansatzpunkte für Korrosionsschäden bilden können. Geschweißte Gehäuse aus Leichtmetall sind besonders teuer.

Anzustreben ist ein möglichst leichtes, aber maßhaltiges Gehäuse, am besten aus Leichtmetall, mit möglichst wenig Fugen.

Diese Forderung erfüllt der im Anspruch 28 beschriebene, erfindungsgemäße Granatwerfer: bei diesem ist der Hauptabschnitt des Gehäuses aus einem Abschnitt eines Hohlprofilmateriales gebildet, das gegebenenfalls spanend nachbearbeitet ist (z.B. Auswurffenster). Das Hohlprofilmaterial ist an seiner Vorderseite durch einen Block verschlossen, in dem das Rohr sitzt und der gegebenenfalls die Längsführung sowie Federführungsstäbe trägt.

Die Rückseite des Hohlprofilmaterial-Abschnitts kann von der Abzugseinrichtung verschlossen sein.
Das Hohlprofilmaterial kann bevorzugt ein stranggepreßtes Hohlprofil vorzugsweise aus Leichtmetall sei, etwa
Duraluminium. Im Inneren kann das Hohlprofilmaterial Längsstege usw. aufweisen, die zur Führung des
Masseverschlusses dienen können, soweit dieser nicht mit dem oben beschriebenen Längsführungsstab geführt ist.

An der Außenseite kann das Hohlprofilmaterial Längsstege, Längsnuten usw. aufweisen, die als Aufnahmen für eine Lafettenmontage, Visierung, Infrarotbeleuchtung u. dgl. dienen können.

Das Hohlprofilmaterial ist bevorzugt gemäß Anspruch 29 ein geschlossenes Kasten- oder Hohlprofil, das den Masseverschluß aufnimmt und durch ein offenes Kasten- oder Hohlprofil nach oben verlängert ist, das den Kurvenhebel aufnimmt und mit einem abnehmbaren Deckel abgedeckt ist. Die längsverlaufende Trennwand zwischen geschlossenem und offenem Hohlprofil weist ein längsverlaufendes, eingefrästes Langloch auf, das von dem am Masseverschluß angebrachten Mitnehmer durchsetzt ist. In dieser Trennwand sitzen auch die Schwenkzapfen für den Kurvenhebel, Umlenkhebel usw..

Der besondere Vorteil dieser Anordnung liegt darin, daß die empfindliche Steuerung gesondert vor Verschmutzung auch durch Pulvergase weitgehend geschützt untergebracht und dennoch ohne weiteres zugänglich ist.

Die Außen- und/oder Innenoberfläche des so gebildeten Gehäuses ist bevorzugt (Anspruch 30) oberflächenbehandelt, um eine Tarnfärbung, die Resistenz gegenüber Korrosion z.B. durch Salzwasser, die verbesserte Abriebfestigkeit und weitere angestrebte Oberflächeneigenschaften zu erreichen.

Als besonders geeignet hat sich die Hartanodisierung der Innen- und Außenoberfläche des Leichtmetallgehäuses erwiesen.

Wie bereits erwähnt, trachtet der Patronengurt, der bei Dauerfeuer ruckartig nachgeführt wird, dazu, heftige, peitschenartige Bewegungen durchzuführen, die zu Funktionsstörungen führen können.

Um diese Bewegungen abzumildern und für einen glatten Einlauf des Patronengurtes in die Waffe zu sorgen, ist gemäß Anspruch 31 eine den Patronengurt von unten her stützende Gurtführungsbühne und gemäß Anspruch 32 eine den Patronengurt von oben her führende Abdeckung vorgesehen, die beide lösbar am Gehäuse der Waffe anbringbar sind.

Während die Gurtführungsbühne und die Abdeckung den Patronengurt unten bzw. oben an die Patronengurt-Einlauföffnung des Waffengehäuses angrenzen, ist diese Öffnung an ihrem vorderen und hinteren Ende gemäß Anspruch 33 je durch eine frei drehbare Patronengurt-Führungsrolle begrenzt. Beide Patronengurt-Führungsrollen weisen einen solchen Durchmesser auf, daß ein Verhaken der Patronen mit den Rollen unmöglich ist. Vorzugsweise sind beide Patronengurt-Führungsrollen kreiszylindrisch und weisen den gleichen Durchmesser auf.

Wie bereits oben mehrfach erwähnt, kann die erfindungsgemäße Einrichtung zum Zuführen und Entgurten des Patronengurtes für die Zuführung von rechts oder von links wahlweise und bevorzugt ohne Verwendung von irgendwelchen Austauschteilen umgestellt werden. Dementsprechend weist das Waffengehäuse gemäß Anspruch 34 zwei einander gegenüberliegende Einführungsöffnungen für den Patronengurt auf.

Diese Einführungsöffnungen sind bevorzugt an beiden Seiten des Gehäuses angeordnet, können aber bei besonderer Verwendung des Granatwerfers etwa in Land-, See- oder Luftfahrzeugen auch an der Ober- und Unterseite des Gehäuses angeordnet sein.

Um eine unnötige Verschmutzung zu vermeiden, ist gemäß Anspruch 35 die gerade nicht benutzte Einführungsöffnung durch eine Wandung oder Abdeckung verschließbar, die bevorzugt als anmontierbare Blechplatte ausgebildet ist, aber auch als einklemmbarer Kunststoffstopfen ausgebildet sein kann.

Die obengenannte Gurtführungsbühne und Abdeckung sind zu ihrer Mittelachse, die jeweils quer zur Waffenlängsachse verläuft, symmetrisch ausgebildet, so daß sie vor jeder der Einführungsöffnungen angebracht werden können.

Um die störungsfreie Zuführung des in einem Patronenkasten verwahrten Patronengurtes noch weiter zu verbessern, sind an diesem Patronenkasten und am Waffengehäuse komplementäre Halterungsmittel ausgebildet, die eine Halterung nahe der oder jeder Einführungsöffnung am Gehäuse umfaßt (Anspruch 36).

Die Abdeckung kann ein ständig am Waffengehäuse angebrachtes Bauelement sein, bildet gemäß Anspruch 37 jedoch bevorzugt einen Teil des Patronenkastens, so daß die Abdeckung, nachdem sie am Waffengehäuse angebracht ist, eine durchgehende, zuverlässige Führung für den Patronengurt vom Patronenkasten in die Waffe bildet.

Bevorzugt ist die Abdeckung am Deckel des Patronenkastens angebracht oder bildet mit diesem ein Teil (Anspruch 38).

Der Patronengurt kann nur dann in die Waffe eingelegt werden, wenn der die Schieber tragende Deckel geöffnet ist und sich der Masseverschluß in seiner hintersten Lage befindet, seiner Auslöselage.

In dieser Auslöselage ist der Masseverschluß allerdings von der Schließfederanordnung belastet und wird lediglich von der Abzugsvorrichtung gehalten.

Wird der Gurt eingelegt oder wird etwa versucht, eine Ladehemmung zu beheben, dann befindet sich die Hand des Schützen im Bewegungsweg des Masseverschlusses. Wird nun der Verschluß versehentlich ausgelöst oder löst er sich etwa infolge eines Fahrbahnstoßes bei dem die Waffe tragenden Fahrzeug, dann ist eine unter Umständen ernste Verletzung der Hand des Schützen zu erwarten. Dies ist umso schwerwiegender, weil der Schütze gerade die verletzte Hand vornehmlich zum Bedienen der Waffe benötigt.

Um diesen Nachteil zu vermeiden, ist bei der erfindungsgemäßen Waffe gemäß Anspruch 39 eine Verschlußsperre vorgesehen, die mit dem Deckel gekoppelt ist und bei geöffnetem Deckel aktiviert ist. Diese Verschlußsperre hält entweder den Masseverschluß in seiner Auslöselage und verhindert sein Abschlagen, auch wenn er versehentlich oder störungsbedingt ausgelöst wurde, oder fängt den nach vorne fahrenden Verschluß auf, bevor er in den Bereich der Patronen-Zuführeinrichtung gelangen und eine dort befindliche Hand verletzen kann.

Diese Verschlußsperre kann mit dem Deckel oder mit dessen Verriegelung zwangsgekoppelt sein, ist aber gemäß Anspruch 40 bevorzugt mit einem Fühler versehen, der feststellt, ob der Deckel sich in seiner geschlossenen Lage befindet oder nicht.

Dieser Fühler kann eine Auslöseeinrichtung ansteuern, ist aber bevorzugt gemäß Anspruch 41 als Tastfinger ausgebildet, der durch eine Feder in seine Sperrlage belastet wird, also in eine Lage, in welcher er die Verschlußsperre wirksam macht. Der Tastfinger ist unmittelbar bewegungsübertragend mit einem Arretierhebel verbunden, der in den Masseverschluß oder in dessen Bewegungsbahn eingreifen kann und und ihn festhalten oder aufhalten kann.

Soweit der Masseverschluß mit einem im Kurvenhebel geführten Mitnehmer ausgestattet ist, ist vorteilhaft gemäß Anspruch 42 der genannte Arretierhebel in die Bewegungsbahn des Mitnehmers beweglich. Der Mitnehmer bildet nämlich ein besonders widerstandsfähiges, vom Masseverschluß abstehendes Bauteil, das auch dann keinen Schaden nimmt, wenn es kraftvoll gegen den Arretierhebel aufläuft.

Um nun auch eine leichte Bauausführung des Arretierhebels zu ermöglichen, greift er gemäß der Ausgestaltung des Anspruchs 43 in eine Aussparung im Kurvenhebel ein. Dieser Kurvenhebel ist als hochbelastetes Bauteil sehr stabil ausgeführt und an einer kräftigen, gehäusefesten Lagerung getragen.

Läuft der Mitnehmer des Masseverschlusses gegen den Arretierhebel an, dann leitet dieser alle von ihm aufgenommenen Kräfte in den ausreichend stabilen Kurvenhebel ein, auf dem er sich in der Aussparung abstützt.

Der Eingriffsvorsprung des Arretierhebels wird hierbei nur wenig belastet, da er alle auf ihn einwirkenden Kräfte weitergibt; somit besteht keine Gefahr, daß der Eingriffsvorsprung oder der Arretierhebel abbricht, sondern es ist gewährleistet, daß die Sperre stets wirksam bleibt.

Die Lage des Eingriffsvorsprunges und der Aussparung im Kurvenhebel ist so gewählt, daß der Masseverschluß erst kurz vor Erreichen der Patronen-Zuführeinrichtung angehalten wird.

Diese Anordnung hat den Vorteil, daß die Sperre, deren Tastfinger sich in den Bereich des Deckels erstreckt, möglichst kurz ausgebildet ist. Außerdem ist es durch das spürbare Abschlagen des Masseverschlusses bis zum Arretierhebel für den Schützen deutlich erkennbar, daß sich der Masseverschluß versehentlich oder störungsbedingt nicht mehr in der Auslöselage befindet, und er kann ihn vor dem Schließen des Deckels wieder in seine Auslöselage bringen, so daß der ungestörte Betrieb der Waffe fortgesetzt werden kann, soweit nicht ein Schaden vorliegt.

Es gibt eine Vielfalt von Sicherungsvorrichtungen, die das unbeabsichtigte Zünden einer Patrone verhindern sollen.

Bei einer Waffe der erfindungsgemäßen Art ist eine solche Sicherung noch wichtiger als bei anderen Waffen, da die Zündung einer Patrone nur dann erfolgen darf, wenn sich der Masseverschluß an einer bestimmten Stelle kurz vor dem Ende seines Bewegungsweges befindet und dort eine bestimmte Geschwindigkeit aufweist.

Eine weitere Aufgabe der Erfindung ist es, eine wirksame Sicherung zu finden.

Diese Aufgabe wird durch die Merkmale des Anspruchs 44 gelöst; demnach sitzt der Schlagbolzen in einer Schlagbolzenhülse, die ihrerseits längsverschieblich im Masseverschluß sitzt und in eine hintere Lage beweglich ist, in der sie den Schlagbolzen daran hindert, zum Zünden einer Patrone aus dem Stoßboden des Masseverschlusses herauszutreten.

Während die Abmessungen, das Material und das Gewicht des Schlagbolzens in engen Grenzen konstruktiv festliegen, gelten solche Einschränkungen für die Schlagbolzenhülse nicht, so daß sie ohne weiteres mit Sicherungseinrichtungen versehen bzw. gekoppelt werden kann.

Diese Schlagbolzenhülse ist gemäß Anspruch 45 so ausgebildet, daß es ausgeschlossen ist, daß der Schlagbolzen eine Patrone zünden kann, wenn sich die Schlagbolzenhülse in ihrer hinteren Stellung befindet.

Die Schlagbolzenhülse ist in besonders vorteilhafter Weise gemäß Anspruch 46 mittels eines Steuerhebels mit einer gehäusefesten Steuerkurve verbunden, so daß die Schlagbolzenhülse praktisch nur im Bereich jener Lage des Masseverschlusses in ihre vorderste Stellung bewegt wird, in welcher die Zündung der Patrone erfolgen soll. Somit ist jegliche unzulässige Zündung ausgeschlossen.

Der Schlagbolzen ist über ein Zwischenteil mit einer Schlagfeder zum Antrieb verbunden, wird in seiner gespannten Stellung eingerastet bzw. arretiert und wird in Abhängigkeit von der Lage des Masseverschlusses ausgelöst.

Wenn durch einen Bruch o. dgl. in einem Funktionteil wie etwa der Schlagfeder der Antrieb des Schlagbolzens durch die Schlagfeder unterbleibt, dann kann sich er beim Abschlagen des Masseverschlusses unkontrolliert bewegen und zu weiteren Schäden führen.

Gemäß Anspruch 47 greift aber das Zwischenteil beim Ausbleiben der Schlagfederbelastung unmittelbar oder mittelbar in die Steuerung der Schlagbolzenhülse so ein, daß sie ständig in ihrer hinteren Lage verbleibt.

Der eingangs genannte, gattungsbildende Granatwerfer weist beiderseits der Bewegungsbahn des Masseverschlusses einen Längsschlitz in jeder der Gehäuse-Seitenwände auf, der jeweils von einem Handgriff durchsetzt ist, der sich quer zur Längsachse der Waffe erstreckt und am Masseverschluß befestigt ist.

Beim Feuern führen die beiden Handgriffe eine Längsbewegung durch; wird diese behindert, dann kann dies zu einer Funktionsstörung führen.

Außerdem kann durch die von den Handgriffen durchsetzten Längsschlitze Schmutz in das Gehäuse gelangen.

Besonders umständlich ist aber der Durchladevorgang: bei diesem muß sich der Schütze über die Waffe beugen und mit einer Hand oder beiden Händen den oder jeden Handgriff ergreifen und kräftig nach hinten ziehen. Da hierbei die Kraft der sehr starken Schließfederanordnung überwunden werden muß, ist ein erheblicher Kraftaufwand erforderlich. Außerdem ist es unmöglich, bei dieser Tätigkeit die Ausrichtung der Waffe auf einer Lafette beizubehalten, da man über den Handgriff oder die Handgriffe die Waffe ihrerseits kräftig nach hinten zieht.

So ist es beispielsweise zwecklos, die ungespannte Waffe auf ein Ziel zu justieren, da die Justierung beim Spannen verlorengeht.

Es ist aber gerade oftmals besonders wesentlich, etwa bei Tageslicht die Waffe auf einen Geländeabschnitt zu richten, etwa einen Geländeeinschnitt oder eine Straße, wo man nachts mit dem Auftreten des Feindes rechnet. Dabei ist es aus Sicherheitsgründen unzulässig, die Waffe ständig gespannt zu halten.

Ferner ist der Schütze etwa beim Auftreten einer Ladehemmung gezwungen, sich über die Waffe zu beugen und somit gefährlich zu exponieren.

Diese Nachteile schränken die Brauchbarkeit des bekannten Granatwerfers in empfindlicher Weise ein.

Diesen Nachteilen wird durch die Merkmale des Anspruchs 48 abgeholfen.

Hierbei ist die Handhabe zum Spannen des Masseverschlusses als ein an der Rückseite des Gehäuses angeordneter Zuggriff ausgebildet, der über ein Gestänge, ein Zugglied o. dgl. mit dem Masseverschluß verbunden ist, so daß der Masseverschluß durch Herausziehen des Zuggriffs mit nach hinten in die Auslöselage mitgenommen wird.

Anschließend wird der Zuggriff wieder vollständig in das Gehäuse eingeschoben, so daß sich der Masseverschluß hin- und herbewegen kann, ohne vom Zuggriff, dessen Gestänge oder dessen Zugglied behindert zu werden.

Diese Ausgestaltung weist keine beim Schuß sich hin- und herbewegende Handhabe auf. Das Gestänge oder Zugglied wird in seiner Längsrichtung durch eine Gehäuseöffnung in der Rückwand des Gehäuses bewegt, nicht in seiner Querrichtung, so daß es stets die von ihnen durchsetzte Gehäuseöffnung voll abdecken kann und somit das Eindringen von Schmutz ausschließt.

Besonders vorteilhaft ist aber der Umstand, daß der Schütze, der zum Spannen des Masseverschlusses die Handhabe bzw. den Zuggriff mit der einen Hand ergriffen hat, sich mit der anderen Hand an der Rückseite des Gehäuses, vorzugsweise an dem dort angebrachten Handgriff, abstützen kann. Somit werden alle Kräfte zwischen den beiden Händen des Schützen wirksam; eine Kraftkomponente, die die Justierung der Waffe stören könnte, kann nach geringer Übung vermieden werden.

Insbesondere aber kann der Schütze beim Spannen des Masseverschlusses hinter der Waffe in Deckung bleiben und muß sich nicht über diese beugen.

Bevorzugt ist gemäß Anspruch 49 eine lösbare Sperre vorgesehen, die die Handhabe bzw. den Zuggriff in der eingeschobenen Lage lösbar festlegt, um zu verhindern, daß sich die Handhabe durch eine Erschütterung o. dergl. löst und über das Gestänge oder das Zugglied in die Bewegung des Masseverschlusses störend eingreift.

Eine besonders zweckmäßige kraftübertragende Verbindung zwischen Handhabe und Masseverschluß ohne zusätzliche Bauteile erfolgt durch die Merkmale des Anspruchs 50. Hierbei sind bevorzugt, wie auch beim gattungsbildenden Granatwerfer, zwei Schließfedern vorgesehen, die jeweils auf einer Federführungsstange geführt sind. Die Schließfedern sind als Druckfedern ausgebildet, sitzen zwischen dem Masseverschluß und dem hinteren Ende des Gehäuses und umgeben jeweils eine Federführungsstange.

Diese Federführungsstangen sitzen aber nicht, wie beim gattungsbildenden Granatwerfer, ortsfest im Gehäuse, sondern sind in ihrer Längsrichtung verschieblich. An ihrer Rückseite tragen sie die Handhabe bzw. den Zuggriff, an ihrer Vorderseite tragen sie eine Mitnehmeranordnung, mit welcher sie den Masseverschluß von vorne her hintergreifen und bei ihrer Rückwärtsbewegung mit nach hinten nehmen.

In ihrer vordersten Stellung, in welcher sie infolge der die Handhabe haltenden, lösbaren Sperre festgelegt sind, tauchen sie mit ihren vorderen Enden bevorzugt in Aufnahmen oder Bohrungen im vorderen Ende des Gehäuses ein und sind somit beim Schuß gegen seitliche Auslenkung stabil gehalten.

Die Mitnehmeranordnung kann von einem überstehenden Ringbund gebildet sein, ist aber bevorzugt als Pufferfeder ausgebildet, die sich mit ihrem vorderen Ende auf einer mit der Federführungsstange fest verbundenen Abstützung, etwa einem Federring, abstützt.

Die beiden Federführungsstangen liegen horizontal nebeneinander in Gehäuse; die Handhabe ist somit als ein horizontaler, sich quer zur Waffenlängsachse erstreckender Griff ausgebildet. Dies ist ergonomisch sehr günstig.

Ferner weist die Waffe bevorzugt an ihrer Rückseite in üblicher Weise zwei aufrechte, nebeneinanderliegende Griffe auf, von denen jedem eine vom Daumen betätigbare Abzugsplatte zugeordnet ist. Beide Abzugsplatten können zu einer einzigen Daumenplatte zusammengefaßt sein.

Beim Spannen des Masseverschlusses ergreift der Schütze mit jener Hand, die er bevorzugt verwendet, den horizontalen Handgriff, mit der anderen Hand den dieser entsprechenden aufrechten Griff. Nun ist die Waffe mit subjektiv geringem Kraftaufwand so aufzuziehen, daß auf die Halterung der Waffe kaum oder keine Kräfte aufgebracht werden.

Die oben genannte Sperre zum lösbaren Halten des horizontalen Handgriffs weist gemäß Anspruch 51 einen integrierten Auslösehebel auf, der beim Ergreifen des Handgriffes zwangsläufig betätigt wird und beim Loslassen des Handgriffes die Sperre wirksam macht. Somit wird ein notwendiger, gesonderter Bedienungsvorgang (Lösen der Sperre) in einem anderen Bedienungsvorgang (Ergreifen bzw. Loslassen des Handgriffs) integriert, so daß die besonders einfache Bedienung sichergestellt ist.

Ein weiteres Problem der bekannten Waffe liegt in der Gefahr ungewollten Abfeuerns durch eine Waffenstörung:

Der Masseverschluß wird von der Abzugseinrichtung lösbar gehalten, wobei sie mittels eines hakenartigen, mittels der Abzugs-Daumenplatte schwenkbaren Schwenkhebels einen Querstollen am Ende des Masseverschlusses hintergreift, der beim Betätigen der Abzugs-Daumenplatte freigegeben wird.

Nun ist die Masse des Masseverschlusses und die Kraft der Schließfederanordnung jeweils groß. Kommen in Längsrichtung wirkende und die Kraft der Schließfeder unterstützende Massenkräfte, etwa durch das harte Anfahren einer Bodenwelle durch das die Waffe tragende Fahrzeug verursacht, hinzu, dann wird die auf den Hakenabschnitt des Schwenkhebels wirkende Kraft sehr groß.

Andererseits darf dieser Schwenkhebel (oder besser ein Paar gleichartiger, nebeneinanderliegender Schwenkhebel) nicht zu schwer sein, damit er nicht seinerseits durch Massenkräfte ungewollt gegen die Kraft der auf ihn einwirkenden Rückstellfeder ausgelöst wird. Diese Rückstellfeder darf ihrerseits nicht zu hart sein, damit ein zielgenaues Abziehen möglich ist. Wegen der aufgezeigten konstruktiven Zwänge ist somit die Gefahr eines Bruchs in kraftaufnehmenden Teilen der Abzugseinrichtung bei einer Waffe der eingangs genannten Gattung größer als etwa bei einer Selbstladepistole. Wird diese Gefahr durch massive Bauausführung des Schwenkhebels gemindert, dann erhöht sich gleichzeitig die Gefahr unbeabsichtigten Abfeuerns durch Massenkräfte, die die Abzugseinrichtung ungewollt betätigen.

Während die letztgenannte Gefahr durch geeignete Sicherungen, die den Schwenkhebel oder ein Element mit besonders hoher Masse in der Abzugseinrichtung festlegen können, gemindert werden kann, ist die Gefahr des Abbrechens des Auslösehebels durch keine Sicherung beherrschbar, die lediglich die Abzugseinrichtung festlegt.

Ausgehend von dieser Problemlage löst die Erfindung diese Schwierigkeiten durch die Merkmale des Anspruchs 52.

Hierbei ist eine zusätzliche, den Masseverschluß unabhängig von der Abzugseinrichtung festhaltende Zusatz-Sicherungseinrichtung vorgesehen.

Versagt die Abzugseinrichtung, dann hält die Zusatz-Sicherungseinrichtung den Masseverschluß in oder nahe seiner Auslöselage fest.

Sollte der oben erwähnte Schwenkhebel gebrochen sein und die Abzugseinrichtung somit unwirksam geworden sein, dann läßt sich notfalls die Waffe noch immer abfeuern, wobei die Zusatz-Sicherungseinrichtung als Abzug benutzt wird. Die Waffe ist somit trotz einer erheblichen Störung nicht gänzlich ausgefallen.

Um diese Not-Bedienungsmöglichkeit zuzulassen, ist die Zusatz-Sicherungseinrichtung gemäß Anspruch 53 mit in der Abzugseinrichtung enthalten, und zwar durch einen eigenen Fanghaken, der vorzugsweise den Abzugsstollen am Masseverschluß wie der mit dem Abzug verbundene Schwenkhebel hintergreift.

Es wäre möglich, den Fanghaken einer eigenen Betätigungsund Arretieranordnung zuzuordnen; nach Anspruch 54 ist er je- doch bevorzugt mit der ohnehin vorhandenen Sicherungseinrichtung gekoppelt, so daß auf einen eigenen Bedienungshandgriff und damit gesonderten Bedienungsvorgang verzichtet werden kann.

Der Fanghaken ist zwangsläufig ein wenig vor der eigentlichen Auslöselage angeordnet, so daß beim Auslösen der Abzugseinrichtung, wenn die Zusatz-Sicherungseinrichtung wirksam ist, sich der Masseverschluß um ein kurzes Stück nach vorne bewegt, in den Fanghaken einfällt und von der eigentlichen Abzugseinrichtung nicht mehr gehalten werden kann.

Wird die Waffe nun entsichert, dann löst sich der Schuß. Um dies zu verhindern, ist gemäß Anspruch 55 eine Vorkehrung getroffen, die das Auslösen des Masseverschlusses, der durch den Fanghaken gehalten wird, nur dann gestattet, wenn er vorher wieder in die Auslöselage zurückbewegt wurde.

Diese Vorkehrung kann in einem tief ausgesparten Fanghaken bestehen, der durch den Abzugsstollen am Masseverschluß, in den er eingreift, so festgehalten wird, daß er nicht in die entsicherte Stellung gelangen kann.

In diesem Fall ist allerdings die oben erwähnte Möglichkeit des Abfeuerns der Waffe mittels der Zusatz-Sicherungseinrichtung nicht gegeben.

Der Gegenstand der Erfindung wird anhand der beigefügten, schematischen Zeichnung beispielsweise noch näher erläutert, in der eine einzige bevorzugte Ausführungsform des erfindungsgemäßen Granatwerfers gezeigt ist. In dieser Ausführungsform sind alle oben aufgeführten Merkmale der Ansprüche vereinigt.

Es wird jedoch ausdrücklich darauf hingewiesen, daß die zusammengehörigen Gruppen dieser Merkmale auch unabhängig von anderen Merkmalsgruppen bei einer Waffe realisiert werden können.

Die Zeichnung zeigt in der einzigen Figur den Längs-Aufriß eines Ausführungsbeispiels eines erfindungsgemäßen Granatwerfers, mit abgenommenem Gehäusedeckel und Zubringerdeckel.

Der gezeigte Granatwerfer besteht im wesentlichen aus einer Gehäusegruppe 100, einer Verschlußgruppe mit Federung und Handhabungseinrichtung 200, einer Zubringergruppe mit Steuerung und einer Abzugseinrichtungsgruppe, 400. Der in den Granatwerfer eingeführte Patronengurt (nicht gezeigt) ist an sich bekannt und bildet als solcher keinen Bestandteil der Waffe.

### Die Gehäusegruppe 100:

Das Hauptteil der Gehäusegruppe ist von einer stranggepreßten Hohlprofilstange 102 gebildet, die im folgenden kurz "Gehäuse" genannt wird und die im wesentlichen einen Querschnitt mit zwei parallelen Seitenschenkeln aufweist, die einstückig an ihrem unteren Ende und etwa in ihrer Mitte durch jeweils einen geraden, rechtwinklig angesetzten Quersteg verbunden sind.

Das Gehäuse 102 weist somit eine linke Gehäusewand, eine rechte Gehäusewand und einen Gehäuseboden auf.

Die Längsmittellinie oder Längsmitte des Gehäuses 102 ist mit 114 bezeichnet.

Das Gehäuse 102 wird durch Ablängen und nachfolgendes spanendes Bearbeiten einer stranggepreßten Hohlprofilstange gebildet, wobei infolge der spanenden Bearbeitung eine vordere, querverlaufende Einfräsung gebildet ist, welche zum Einführen des Patronengurtes dient, mit einer rechten Einführöffnung und einer linken Einführöffnung, ferner eine in der rechten Gehäusewand ausgearbeitete Auswurföffnung 120, durch welche abgeschossene Patronenhülsen, Exerzierpatronen oder Patronenversager aus dem Gehäuse ausgeworfen werden, und eine längsverlaufende Einfräsung im oberen Quersteg, so daß von diesem ein, rechter Gehäusesteg und linker Gehäusesteg gebildet ist; an jeder der einander zugewandten Kanten der beiden Gehäusestege ist jeweils eine Stahlleiste mit einer Steuerkurve angebracht, und zwar die Steuerkurve für die Schlagbolzenhülse an der rechten Kante und die Steuerkurve zum Steuern des Abschlagens des Schlagbolzens an der linken Kante.

Dort, wo die von den beiden Gehäusestegen begrenzte Aussparung im oberen Quersteg nicht erforderlich ist, bleibt dieser stehen, etwa bei der Brücke 144.

Das Gehäuse 102 ist hartanodisiert, um eine geeignete Einfärbung (Tarnfarbe) und Oberflächengüte (Abriebbeständigkeit und Gleitverhalten) zu erhalten.

Im vorderen Ende des Gehäuses 102 ist ein Stahlblock 104 fest angebracht, der das auf die Längsmitte 114 zentrierte Rohr 106 mit dem Patronenlager 108 trägt.

Der Stahlblock 104 weist unterhalb und beiderseits des Rohres 106 jeweils eine nach hinten offene Sack-Aufnahmebohrung 134 auf, die jeweils von einem Federführungsstab 214 durchsetzt ist und eine auf diesem sitzende und abgestützte Pufferfeder 218 aufnimmt.

Die Pufferfeder reicht nach hinten bis in die vergrößerte Mündung der Aufnahmebohrung 134; diese Mündung ist so bemessen, daß sie jeweils einen Vorsprung 204 des Verschlußträgers 228 des Masseverschlusses 202 aufnehmen kann, wenn sich dieser bis ganz nach vorne bewegt (beim Abschlagen ohne Patrone).

In den Boden der Sackbohrung 134 mündet eine abgesetzte Abstütz- und Aufnahme-Durchgangsbohrung ein, in der der mit einer Führungsringwulst und einem Endzapfen ausgebildete Federführungsstab 214 im wesentlichen spielfrei aufgenommen ist. Hierbei ist das freie, vordere Ende des Endzapfens abgerundet, so daß der Federführungsstab, wenn er nach vorne in die Aufnahmebohrung 134 bewegt wird, sich selbst ausrichten kann.

In der Mitte zwischen den beiden Aufnahmebohrungen ist das Gehäuse 102 der Länge nach von einem Paß-Rundstab 132 durchsetzt, der im Stahlblock 104 befestigt ist und der den Masseverschluß 202 bei seiner Bewegung führt.

An der Rückseite des Gehäuses 102 ist dieses durch eine Endabdeckung 110 verschlossen, in der zwei Führungen 136 für die Federführungsstäbe 214 sitzen und in der der Paß-Rundstab 132 abgestützt ist.

Die Endabdeckung trägt einen Teil der Abzugseinrichtungsgruppe 400 und ist zusammen mit dieser und der Verschlußgruppe 200 nach hinten abnehmbar.

Die Oberseite des Gehäuses 102 ist von einem abnehmbaren Gehäusedeckel 112 abgedeckt, der sich von der Endabdeckung 110 bis etwa zur Brücke 144 erstreckt.

Etwa in der Mitte der Länge des Gehäuses 102 ist an der Innenseite des Gehäusebodens eine Ratschenklinke 142 um eine Querachse schwenkbar angebracht und durch eine Federung (nicht gezeigt) so belastet, daß sie danach trachtet, eine im wesentlichen aufrechte Lage einzunehmen.

Am Gehäuse 102 sind noch weitere, hier im einzelnen nicht dargestellte Teile befestigt, etwa ein Ausstoßer an der Innenseite der linken Gehäusewand, eine Montage für eine Visiereinrichtung an der Außenseite der linken Gehäusewand, jeweils eine Halterung für einen Patronenkasten außen an der linken oder rechten Gehäusewand im Bereich der linken und rechten Einführöffnung, eine Halterung zum Anbringen einer Lafette außen an der linken und rechten Gehäusewand und/oder am Gehäuseboden usw.

Außerdem ist am hinteren Ende außen an der linken und rechten Gehäusewand jeweils ein oberer und unterer, sich nach hinten und außen erstreckender Haltebügel angebracht; die Enden der übereinanderliegenden Haltebügel sind jeweils durch einen insgesamt vertikalen linken bzw. rechten Handgriff 148 verbunden.

Das Ergreifen eines oder beider Handgriffe 148 erlaubt das Richten und Feuern des Granatwerfers in üblicher Weise.

Schließlich ist an der Rückseite der linken Gehäusewand unten und außen ein sich nach hinten erstreckender Fortsatz angebracht, der an seinem hinteren Ende einen einwärts weisenden Rastvorsprung aufweist, aber insgesamt so angebracht ist, daß er das Entnehmen und Einsetzen der Verschlußgruppe 200 nicht behindert.

### Die Verschlußgruppe 200:

Die Verschlußgruppe 200 weist einen Masseverschluß 202 auf, der aus einem zur Längsmitte 114 koaxialen Verschlußkopf 224 und einem zu dieser parallelen Verschlußträger 228 gebildet ist, die übereinanderliegen und an ihrer Rückseite einstückig miteinander verbunden sind.

Der Verschlußkopf 224 weist an seiner Vorderseite einen Stoßboden auf, der an der rechten Seite von einem üblichen, abgefederten, nach vorne überstehenden Auszieher begrenzt ist.

Diesem gegenüberliegend ist auch an der linken Seite ein Auszieher (nicht gezeigt) angeordnet, um auch bei Erschütterungen der Waffe ein störungsfreies Ausziehen der Patronenhülse durch den vom Patronengurt eingenommenen Bereich hindurch bis vor die Auswurföffnung 120 sicherzustellen; dieser linke Auszieher wird beim Verschlußrücklauf durch einen gehäusefesten Anschlag geöffnet und gibt den Rand der ausgezogenen Patronenhülse frei, kurz bevor dieser gegen den ebenfalls gehäusefesten Ausstoßer aufläuft.

Der Verschlußkopf 224 weist koaxial zur Längsmitte 114 eine Axialbohrung auf, die als nach hinten offene Sackbohrung ausgebildet ist, deren Boden in üblicher Weise von einem Durchtrittskanal für die Spitze des Schlagbolzens durchsetzt ist.

Diese Axialbohrung nimmt die schon oben angesprochene Schlagbolzenhülse, den Schlagbolzen und dessen Schlagfeder (nicht gezeigt) auf.

Der Verschlußträger 228 weist drei Bohrungen auf: eine (nicht gezeigte) Paßbohrung, die dazu eingerichtet ist, im wesentlichen spielfrei auf dem Paß-Rundstab 132 zu gleiten, und zwei nach hinten offene Schließfeder-Aufnahme-Sackbohrungen 206, die zu jeweils einer der Aufnahmebohrungen 134 koaxial sind.

Der Boden dieser Schließfeder-Aufnahme-Sackbohrungen 206 ist jeweils von einer kleineren Bohrung durchsetzt, durch die sich jeweils ein Federführungsstab 214 hindurch erstreckt.

Auf den hinteren Abschnitt eines jeden Federführungsstabes 214 ist jeweils eine Schließfeder 234 aufgeschoben, die als wendelförmige Druckfeder ausgebildet ist.

Jede dieser Schließfedern stützt sich vorne gegen den Boden der zugehöri-gen Schließfeder-Aufnahmebohrung 206 und hinten gegen die Federstabführung 136 ab.

An der Vorderseite des Verschlußträgers 228 ist der schon oben erwähnten Vorsprung 204 ausgebildet.

Wie bei der Erläuterung der Gehäusegruppe 100 dargelegt, erstrecken sich die Federführungsstäbe 214 im schußbereiten Zustand des Granatwerfers nach vorne bis in die entsprechenden Ausbildungen einer zugehörigen Aufnahmebohrung 134 im Stahlblock 104, in der dann auch eine auf den Federführungsstab 214 aufgeschobene Pufferfeder 218 aufgenommen ist.

Diese Pufferfeder 218 kann sich entweder unmittelbar gegen den Boden der Aufnahmebohrung 134 oder gegen einen Radialvorsprung des Federführungsstabes 214 abstützen.

Beim Zurückziehen des Federführungsstabes 214 wird die Pufferfeder 218 entweder durch die vor dieser am Federführungsstab 214 ausgebildete Führungsringwulst oder durch ihre Abstützung am Federführungsstab 214 selbst mitgenommen.

Die beiden Federführungsstäbe 214 erstrecken sich durch die Federstabführungen 136 hindurch nach hinten und sind dort durch einen Spanngriff 216 fest miteinander verbunden, der sich unter den unteren Enden des rechten und linken Handgriffs 148 quer bzw. horizontal erstreckt.

Um den Verschluß 202 zu spannen, wird der Spanngriff 216 hinlänglich weit horizontal nach hinten aus dem Gehäuse 102 herausgezogen und wieder bis zum Anschlag nach vorne zurückgeschoben. Dabei ergreift die eine Hand des Schützen den dieser entsprechenden Handgriff 148 zur Abstützung, während die andere Hand den Spanngriff 216 betätigt. Somit ist ein Spannen der Waffe möglich, ohne daß sich der Schütze über die Waffe beugen muß und ohne daß auf die Waffe Kräfte aufgebracht werden, die geeignet sind, ihre gegebenenfalls vorher erfolgte Einjustierung auf ein Ziel zu beeinträchtigen.

Im Bereich des linken Endes des Spanngriffes 216 ist an diesem ein um eine vertikale Achse schwenkbarer, federnd nach außen gedrückter Auslösehebel angebracht, der so angeordnet ist, daß er bei voll nach vorne geschobenem Spanngriff 216 den Rastvorsprung des Gehäuses 102 sperrend hintergreift. Dabei sind die einander zugewandten Kanten von Rastvorsprung und/oder Auslösehebel so abgeschrägt, daß sie ineinander einrasten, wenn sie gegeneinander bewegt werden.

Der Auslösehebel ist mit einer (nicht gezeigten) Verlängerung versehen, die'so am Spannhebel 216 angeordnet ist, daß sie bei dessen Ergreifen ohne weiteres mit ergriffen werden kann, so daß die vom Rastvorsprung und dem Auslösehebel gebildete lösbare Sperre gelöst wird und den Spannvorgang nicht behindert.

Wird der Spanngriff 216 dagegen bis ganz nach vorne geschoben und losgelassen, dann tritt diese lösbare Sperre in Eingriff und verhindert jegliches unerwünschte Freikommen des Spannngriffs 216.

Der Verschlußkopf 224 trägt ferner hinten an seiner Oberseite einen mittig angeordneten Kurvenhebel-Mitnehmer 222, der bevorzugt als eine um eine vertikale Achse drehbare, gehärtete Rolle ausgebildet ist.

An der Rückseite des Masseverschlusses 202 ist ferner ein Abzugsstollen 230 angeordnet, der als querverlaufende, sich nach oben erstreckende Leiste ausgebildet ist, deren Oberkante knapp unter der Längsmitte 114 liegt und deren Vorderseite eine im wesentlichen vertikal abfallende Querfläche bildet.

Der Abzugsstollen 230 ist so ausgebildet, daß er von einer Nase am vorderen Ende eines in der Abzugseinrichtung 402 um eine horizontale Achse schwenkbar gelangerten Abzugshebels 404 von oben her umgriffen wird. Wird der Abzugshebel 404 mit seiner Nase nach oben weggeschwenkt, dann gibt er den Abzugsstollen 230 und damit den Masseverschluß 202 frei, so daß dieser unter der Wirkung der Schließfedern 234 nach vorne schnellen kann.

Oberhalb des Abzugsstollens 230 ist ein hakenartiger, nach vorne offener und von oben her ergreifbarer Fangvorsprung ausgebildet.

An der Unterseite des Verschlußträgers 228 ist eine Reihe in Längsrichtung hintereinanderliegender Ratschenzähne 226 angeordnet, deren vordere Zahnflanken sich vertikal erstrekken, deren Zahnspitzen horizontal abgeflacht sind und deren hintere Zahnflanken gegenüber der Horizontalen jeweils nur um einen sehr flachen Winkel, etwa 10°, geneigt sind.

Der Grund zwischen der hinteren Zahnflanke eines vorderen Ratschenzahnes 226 und der vorderen Zahnflanke eines nachfolgenden Ratschenzahnes 226 ist horizontal abgeflacht.

Der vertikale Abstand zwischen den Ratschenzähnen 226 und der am Gehäuse 102 schwenkbar angebrachten Ratschenklinke 142 ist so bemessen, daß die Ratschenklinke 142 sich unter den Ratschenzähnen 226 nur bis zu einer solchen Schräglage aufrichten kann, daß sie imstande ist, dann, wenn sie nach hinten gekippt ist, sperrend gegen eine der vorderen Zahnflanken anzuliegen, dagegen dann, wenn sie nach vorne gekippt ist, die Ratschenzähne 226 unbehindert über sich weggleiten zu lassen.

Die Länge der zahnstangenartigen Reihe von Ratschenzähnen 226 und damit des Verschlußträgers 228 ist so bemessen, daß diese Reihe voll über die Ratschenklinke 142 nach vorne oder hinten hinweggelaufen ist, wenn sich der Masseverschluß 202 in seiner vordersten oder hintersten Lage befindet.

In jeder dieser Lagen ist es der Ratschenklinke 142 somit gestattet, sich durch Federwirkung vollständig aufzurichten, so daß sie beim Rücklauf des Masseverschlusses 202 nach hinten, beim Vorlauf dagegen nach vorne gekippt wird.

In der gezeigten Stellung befindet sich der Masseverschluß 202 in seiner Auslöselage, in der er durch den Eingriff des Abzugshebels 404 in den Abzugsstollen 230 in seiner Lage festgehalten wird. Diese Auslöselage befindet sich ein wenig vor dem hintersten Ende des Rücklaufes, wo es der Ratschenklinke gestattet ist, sich ganz aufzurichten. Nun, in der Auslöselage, liegt-das vordere Ende der zahnstangenartigen Reihe von Ratschenzähnen 226 von hinten her gegen die Ratschenklinke 142 an und kippt sie nach vorne.

Wird nun der Masseverschluß 202 freigegeben, dann läuft er unbehindert über die Ratschenklinke 142 hinweg, bis er in seine vorderste Lage gelangt. Hier richtet sich die Ratschenklinke 142 hinter der zahnstangenartigen Reihe wieder auf und wird beim Rücklauf nach hinten gekippt.

Wird nun aus irgendeinem Grund der Rücklauf unterbrochen, etwa weil eine Patrone mit ungenügendem Rückstoß abgefeuert wurde oder der Schütze beim Spannen des Masseverschlusses 202 behindert wurde, so daß dessen Rückwärtsbewegung schon vor der Auslöselage abgebrochen wird, dann kann der Masseverschluß 202 nicht nach vorne schnellen. Dies ist erst dann möglich, wenn die Rücklaufbewegung mittels des Spanngriffs 216 vervollständigt wurde.

Somit wird ein unerwünschtes Abfeuern verhindert, das möglicherweise, etwa beim Loslassen des Spanngriffes 216, stattfinden könnte, weil in der dann erreichten Stellung des Masseverschlusses 202 (vor der Auslöselage) der Abzugshebel 404 noch nicht in den Abzugsstollen 230 eingreifen und den Masseverschluß 202 festhalten kann.

Das Aufhalten des Masseverschlusses 202 in einer Lage vor der Auslöselage ist bei vielen Waffen, etwa den meisten Maschinenpistolen oder Maschinengewehren, zweckmäßig, ist aber bei der dargestellten Waffe darüber hinaus deshalb von grundlegender Bedeutung, weil bei dieser Waffe die Zündung der Patrone 502 nicht erst dann erfolgt, wenn sie voll in das Patronenlager 108 eingeführt wurde, sondern schon einen kurzen, genau festgelegten Zeitraum vorher, wenn sich Patrone 502 und Masseverschluß 202 in voller Bewegung befinden, wobei in an sich bekannter Weise die dann aufgebrachte kinetische Energie zum Abfangen eines Teiles des Rückstoßes dient, der durch den Abschuß der Patrone 502 entsteht.

Da aber die Patrone 502 nicht voll ins Patronenlager 108 eingeführt werden kann, sondern um einen beträchtlichen Abstand (axiale Länge des Gurtgliedes) aus dem Patronenlager 108 herausragt, wenn sie gezündet wird, werden die genaue Lage des Masseverschlusses 202 und dessen eng tolerierte Geschwindigkeit jeweils zum Zündzeitpunkt zu höchst kritischen Werten. Das beschriebene Ratschengesperre 142, 226 sorgt dafür, daß die Geschwindigkeit des Masseverschlusses 202 sich beim Zünden der Patrone 502 zuverlässig innerhalb der zulässigen Toleranz befindet.

## Patentansprüche

1. Selbstlade-Granatwerfer mit
- einer Patronengurt-Zuführeinrichtung, die mittels in den Patronengurt eingreifender Klinken eine Patrone (502) bevorzugt in horizontaler Richtung bis vor ein Patronenlager (108) fördert, wobei die Klinken, von zwei in einem aufklappbaren Zubringerdeckel angeordneten, gegenläufigen, quer zur Schußrichtung beweglichen Schiebern getragen sind und nach unten abstehen,
- einem Masseverschluß (202), der aus einer Auslöselage durch die Federkraft einer, vorzugsweise zweier Schließfedern (234) nach vorne längs eines Bewegungsweges gegen das Patronenlager (108) läuft und dazu eingerichtet ist, bei diesem Vorlauf die von den Klinken geförderte Patrone (502) aus dem Patronengurt in das Patronenlager (108) zu schieben sowie nach deren Zündung durch den entstehenden Rückstoß den Bewegungsweg in einem Rücklauf zurückzulegen und dabei die Schließfeder(n) (234) zu spannen,
- einer mit dem Masseverschluß (202) und den Schiebern verbundenen Steuerung, die den Vor- und Rücklauf des Masseverschlusses (202) in die hierzu quergerichtete Wechselbewegung der Schieber umsetzt,
- einer Zündeinrichtung mit einem Schlagbolzen, der - vorzugsweise durch eine Schlagfeder - gespannt und in gespanntem Zustand durch eine Arretierung gehalten ist, wobei die Arretierung gelöst und die Patrone (502) gezündet wird, bevor der Masseverschluß (202) seinen Vorlauf beendet hat, aber erst, nachdem die Patrone weit genug in das Patronenlager eingeführt ist, um dem Gasdruck des Abschusses standzuhalten, und
- einem mit dem Patronenlager verbundenen, sich längs des Bewegungsweges erstreckenden und diesen mindestens teilweise umschließenden Gehäuse (102), dessen Längsmitte die Mittelachse des Patronenlagers aufnimmt,
- wobei zwischen dem Masseverschluß (202) und dem Gehäuse (102) eine Sperre (142, 226) angeordnet ist, die nach jedem Rücklauf, entweder infolge des Rückstoßes oder der Handbetätigung, den nachfolgenden Vorlauf erst dann gestattet, wenn der Masseverschluß (202) eine Mindest-Spannlage erreicht hat.

2. Granatwerfer nach Anspruch 1, dadurch gekennzeichnet, daß die Mindest-Spannlage des Masseverschlusses (202) an oder unmittelbar vor dessen Auslöselage liegt.

3. Granatwerfer nach einem der Ansprüche 1 oder 2, dadurch gekennzeichnet, daß die'Sperre als Ratschengesperre (142, 226) ausgebildet ist, mit einer Reihe von schrägverzahnten Ratschenzähnen (226), die längs des Masseverschlusses (202) oder des Gehäuses (102) angeordnet sind, und mindestens einer mit den Ratschenzähnen (226) in Eingriff bringbaren Ratschenklinke (142), die beim Vorlauf des Masseverschlusses (202) in sperrenden Eingriff mit den Ratschenzähnen (226) tritt, aber durch Vorbeilaufen an einer Nase ausschaltbar ist, die an einer Stelle angeordnet ist, die der Mindest-Spannlage entspricht.

4. Granatwerfer nach Anspruch 3, dadurch gekennzeichnet, daß die Ratschenklinke (142) durch eine Feder in eine Lage zwangsbeweglich ist, in welcher sie sich quer zum Bewegungsweg des Masseverschlusses (202) erstreckt, und daß die Ratschenzähne (226) auf einer gegenüber dem sie tragenden Masseverschluß (202) oder Gehäuse (102) überstehenden, zahnstangenartigen Anordnung sitzen, deren Anfang und Ende beim Rück- bzw. Vorlauf sich über die Ratschenklinke (142) hinausbewegt und so jeweils deren Aufrichten ermöglicht.

## Claims

1. A self-loading grenade launcher with
- a cartridge belt feed device, which by means of pawls engaging in the cartridge belt conveys a cartridge (502) preferably in the horizonal direction until it is in front of a cartridge chamber (108), wherein the pawls are borne by two counteracting slides disposed in a hinged cover and movable transversely to the firing direction, and project downwards,
- an inertia bolt (202), which from a release position runs forward along a path under the spring force of one, preferably two return springs (234) towards the cartridge chamber (108) and is set up to slide the cartridge (502) conveyed by the pawls, during this forward movement, out of the cartridge belt into the cartridge chamber (108) and also after its firing by the resultant blowback to run back along the path in a recoil movement and in so doing to tension the return spring(s) (234),
- a control system connected to the inertia bolt (202) and the slides, which converts the forward and reoil movement of the inertia bolt (202) into the alternating movement of the slides directed transversely thereto,
- a firing device with a firing pin which is tensioned - preferably by a main spring - and is held by a detent in the tensioned state, whereby the detent is released and the cartridge (502) is fired before the inertia bolt (202) has completed its forward travel, but only after the cartridge has penetrated far enough into the cartridge chamber to withstand the gas pressure of the discharge, and
- a housing (102) connected to cartridge chamber, extending along the path of movement and at least partially surrounding it, the longitudinal axis of which houses the central axis of the cartridge chamber,
- wherein a stop (142, 146) is disposed between the inertia bolt (202) and the housing, which stop after each recoil, either as a result of the blowback or of the manual operation, only permits the subsequent forward travel if the inertia bolt (202) has reached a minimum tensioning position.

2. A grenade launcher according to Claim 1,
**characterised in that** the minimum tensioning position of the inertia bolt (202) lies on or directly in front of its release position.

3. A grenade launcher according to one of Claims 1 or 2,
**characterised in that** the stop is constructed as a ratchet mechanism (142, 226), with a series of helical ratchet teeth (226), which are disposed along the inertia bolt (202) or the housing (102), and at least one ratchet pawl (142) which can be brought into engagement with the ratchet teeth (226), which upon the forward travel of the inertia bolt (202) enters into locking engagement with the ratchet teeth (226), but can be disconnected by moving past a lug which is disposed at a site corresponding to the minimum tensioning position.

4. A grenade launcher according to Claim 3,
**characterised in that** the ratchet pawl (142) is automatically moved by a spring into a position in which it extends transversely to the path of movement of the inertia bolt (202),
**and in that** the ratchet teeth (226) are seated on a rack-type arrangement that projects in relation to the inertia bolt (202) or housing (102) bearing them, the beginning and end of which move during the recoil and forward movement over the ratchet pawl (142) and thus enables their raising.

## Revendications

1. Lance-grenades à chargement automatique comportant
- un dispositif d'introduction de la bande de cartouches qui, au moyen de cliquets venant en prise dans la bande de cartouches, transporte une cartouche (502), de préférence en direction horizontale, jusqu'en avant d'un magasin à cartouche (108), dans lequel les cliquets sont portés par deux coulisseaux qui sont disposés dans un couvercle d'amenée rabattable en ouverture, qui se déplace à contre-allure et transversalement à la direction du tir, et dans lequel les cliquets dépassent vers l'arrière,
- une culasse (202) qui, depuis une position de départ, est sous l'action de la force d'un ressort de fermeture, de préférence de deux ressorts de fermeture (234), court vers l'avant le long d'un trajet en direction du magasin à cartouche (108) et est organisée pour, lors de ce mouvement vers l'avant, pousser hors de la bande de cartouches, dans le magasin à cartouche (108), la cartouche (502) amenée par les cliquets, ainsi que, après la mise à feu de cette cartouche, reparcourir en arrière son trajet sous l'action du recul qui se produit et y mettre sous contrainte le (les) ressort (s) de fermeture (234),
- un organe de commande qui est relié à la culasse (202) et aux coulisseaux et qui convertit le mouvement de la culasse (202) vers l'avant et vers l'arrière en le mouvement alterné, orienté transversalement, des coulisseaux,
- un dispositif de mise à feu comprenant un percuteur qui est armé - de préférence sous l'action d'un ressort d'armement - et qui est maintenu à l'état armé par un verrou, le verrou étant déverrouillé et la cartouche (502) mise à feu avant que la culasse (202) ait terminé son mouvement en avant, mais seulement après que la cartouche soit suffisamment introduite dans le magasin à cartouche pour résister à la pression des gaz de la mise à feu,
- un carter (102) qui est relié au magasin à cartouche, s'étend le long du trajet de la culasse, enserre au moins partiellement ce magasin à cartouche et dont l'axe longitudinal correspond à l'axe du magasin à cartouche,
- dans lequel, entre la culasse (202) et le carter (102) est disposé un verrou (142, 226) qui, après chaque retour de la culasse en arrière, soit par suite de l'effet de recul soit par manoeuvre à la main, n'autorise le mouvement vers l'avant suivant qu'après que la culasse (202) a atteint une position minimale d'armement.

2. Lance-grenades selon la revendication 1, caractérisé par le fait que la position minimale d'armement de la culasse (202) se situe à sa position de départ ou immédiatement en avant de cette position de départ.

3. Lance-grenades selon l'une des revendications 1 ou 2, caractérisé par le fait que le verrou est conçu sous forme d'un verrou à cliquet (142, 226) comprenant une rangée de dents d'encliquetage (226), à denture oblique, qui sont disposées le long de la culasse (202) ou du carter (102) et au moins un cliquet (142) qui peut engrener avec les dents d'encliquetage (226), qui, lors du mouvement de la culasse (202) en avant, vient en prise, formant verrouillage, avec les dents d'encliquetage (226), mais peut être mis hors prise en passant devant un talon disposé en une position qui correspond à la position minimale d'armement.

4. Lance-grenades selon la revendication 3, caractérisé par le fait que le cliquet (142) est obligatoirement mis, par un ressort, dans une position dans laquelle il s'étend transversalement par rapport au trajet de la culasse (202) et que les dents d'encliquetage (226) se trouvent sur un dispositif en forme de crémaillère qui déborde par rapport à la culasse (202) ou au carter (102) qui les porte et dont, lors du mouvement vers l'arrière ou vers l'avant, le début et la fin passent au-delà du cliquet (142), ce qui permet chaque fois à ce cliquet de se redresser.
